# EUROPÄISCHE PATENTSCHRIFT

(11) **EP 4 226 897 B1**
(45) Veröffentlichungstag und Bekanntmachung des Hinweises auf die Patenterteilung: **03.07.2024**
(21) Anmeldenummer: 22156256.4
(22) Anmeldetag: 11.02.2022
(51) Int. Cl.: A61F 2/30, A61F 2/32, A61F 2/38

(54) **AUGMENTATIONSVORRICHTUNG**
AUGMENTATION DEVICE
DISPOSITIF D'AUGMENTATION

(43) Veröffentlichungstag der Anmeldung: 16.08.2023
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 3 909 549
- WO-A1-2022/008441
- US-A- 4 519 101
- US-A1- 2018 200 061
- US-A1- 2019 015 215
- US-B2- 10 932 913

## Beschreibung

Die Erfindung betrifft eine Augmentationsvorrichtung umfassend einen ringförmigen Konus, der einen Kanal umgibt, welcher sich axial entlang einer Längsachse der Augmentationsvorrichtung von einem proximalen Konusende zu einem distalen Konusende erstreckt,
wobei ein Außendurchmesser des Konus vom proximalen Konusende in Richtung des distalen Konusende abnimmt.

Gegenstand der Erfindung ist insbesondere eine Augmentationsvorrichtung zum Einsatz bei Gelenkendoprothesenoperationen, insbesondere Revisionsgelenkendoprothesenoperationen für Knie- oder Hüftgelenkte.

Die erfindungsgemäße Augmentationsvorrichtung ist dazu geeignet, debridiertes Knochengewebe zu verstärken oder gar teilweise zu ersetzen und so eine sichere und stabile Verankerung einer Gelenkendoprothese, insbesondere einer Revisionsendoprothese, in einem Knochenkanal zu ermöglichen. Zudem dient die erfindungsgemäße Augmentationsvorrichtung insbesondere einer gleichmäßigen Krafteinleitung in das umgebende Knochengewebe im Zuge einer Implantation einer Gelenkendoprothese, insbesondere einer Revisionsgelenkendoprothese. Die Augmentationsvorrichtung kann insbesondere unter Vermeidung größerer Verluste an Knochengewebe explantiert werden.

### Hintergrund der Erfindung

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte, beziehungsweise nekrotische Gewebe abgetragen. Dieses Abtragen von infiziertem/nekrotischem Gewebe wird als Debridement bezeichnet. Dabei kann es im Bereich der entfernten Gelenkendoprothese zu einem substanziellen Verlust an Knochengewebe, insbesondere bei vorgeschädigter Knochengewebesubstanz, beispielsweise durch Osteoporose, kommen, was zu Problemen bei der Verankerung einer Revisionsgelenkendoprothese führen kann. Um das verbliebene Knochengewebe zu verstärken oder teilweise gar zu ersetzen und eine möglichst gleichmäßige Krafteinleitung in das verbliebene Knochengewebe beim Einsatz der Revisionsgelenkendoprothese zu gewährleisten, besteht eine mögliche Behandlungsoption in einem Einsatz einer Augmentationsvorrichtung in Form eines Metallkonus. Dazu wird das infizierte Knochengewebe abgetragen und die dadurch entstandene Kavität durch Einsetzten der Augmentationsvorrichtung aufgefüllt. Die konusförmige Ausgestaltung der Augmentationsvorrichtung dient dazu, einen Schaft einer Prothese, insbesondere einer Gelenkendoprothese oder Revisionsgelenkendoprothese, in einen axial verlaufenden Kanal der Augmentationsvorrichtung aufzunehmen und dort, beispielsweise unter Einsatz von Knochenzement, zu verankern. Die Augmentationsvorrichtung selbst ist so gestaltet, dass sie in die durch das Debridement entstandene Kavität eingebracht werden kann. Um ein Einwachsen von sich neu bildendem Knochengewebe zu ermöglichen und so die Augmentationsvorrichtung fest mit dem zu behandelnden Knochen zu verbinden, weisen die Metallkonen an ihrer Mantelfläche eine strukturierte, beispielsweise eine aufgeraute oder Spongiosa-artige, Oberflächenstruktur auf. Eine derartige Augmentationsvorrichtung ist beispielsweise in der Patentschrift US 8,506,645 B2 beschrieben.

Um den unterschiedlichen anatomischen Begebenheiten der Patienten gerecht zu werden, werden im Markt Augmentationsvorrichtungen mit unterschiedlicher Größer, insbesondere mit unterschiedlichen Außendurchmessern, angeboten.

Bei septischen Revisionen kann es, trotz sorgfältigem Debridieren im Zuge der septischen Revision, zu einem Wiederaufflammen der zuvor behandelten Infektion kommen, was eine Explantation der Augmentationsvorrichtung samt Revisionsgelenkendoprothese erforderlich macht. Da die Metallkonen aufgrund ihrer strukturierten Mantelflächen mit dem Knochengewebe verwachsen sind, ist eine Explantation derselben nur unter Schwierigkeiten möglich. Häufig müssen die eingewachsenen Metallkonen mit Hilfe von Meißeln vom Knochengewebe getrennt werden und aufgrund der starren Struktur der Konen axial aus dem knöchernen Implantatlager herausgerissen werden. Beides kann zu erheblichen Verlusten an Knochengewebe führen, was die Implantation einer neuen Prothese zusätzlich erschwert. Bei massiven Knochengewebeverlusten verbleibt nur die Implantation einer Tumorgelenkendoprothese als Behandlungsoption, wenn die biomechanische Funktion des Gelenks weitgehend erhalten bleiben soll.

In der Patentschrift EP 3 319 558 B1 wird eine Augmentationsvorrichtung beschrieben, deren Außendurchmesser in bestimmten Grenzen variiert werden kann. Dazu weist die Augmentationsvorrichtung einen ringförmigen Konus auf, welcher zumindest ein axial verlaufendes Biegegelenk aufweist. Durch das zumindest eine Gelenk lässt sich der Konus durch einen von außen einwirkenden Druck zusammendrücken, was den Außendurchmesser der Augmentationsvorrichtung verringert. Durch das Zusammendrücken des Konus wird eine Explanation der Augmentationsvorrichtung aus dem Patienten erleichtert.

Als nachteilig ist bei einer Verwendung von Gelenken, insbesondere Biegegelenken anzusehen, dass die Augmentationsvorrichtung nur in geringem Umfang zusammendrückbar ist, um den Außendurchmesser zur erleichterten Explantation zu verringern. Zwar ist die Trennung von Konus und Knochen an der Stelle des Gelenks vereinfacht, allerdings verbleibt der vom Knochen gelöste Teil der Augmentationsvorrichtung innerhalb des Knochenkanals, was ein Lösen weiterer Teile der Augmentationsvorrichtung vom Knochen erschwert. Zudem führt das Biegen der Gelenke zu, insbesondere scharfkantigen, Einknickungen des Metallkonus, welche die Verletzungsgefahr für den Chirurgen und den Patienten erhöht. Der eingeknickte Konus erschwert zudem aus sterischen Gründen ein Hantieren innerhalb des Knochenkanals.

Beispiele für Augmentationsvorrichtungen mit an der Mantelfläche angeordneten Elementen sind durch EP 3 909 549 A1 und US 2019/015215 A1 offenbart.

Wünschenswert ist daher eine Augmentationsvorrichtung, welche im implantierten Zustand ein Aufwachsen, bevorzugt ein Einwachsen, von Knochengewebe auf oder an ihrer Oberfläche erlaubt und trotzdem einfach, schnell, sicher sowie mit lediglich geringem Verlust an Knochengewebe explantierbar ist.

### Aufgaben

Eine Aufgabe der vorliegenden Erfindung ist es, einen oder mehrere der sich aus dem Stand der Technik ergebenen Nachteile zumindest teilweise zu überwinden.

Insbesondere soll eine Augmentationsvorrichtung bereitgestellt werden, welche ein Aufwachsen, bevorzugt ein Einwachsen, von Knochengewebe auf oder an einer Oberfläche der Augmentationsvorrichtung erlaubt und welche gleichzeitig ein einfaches und sicheres Explantieren bei lediglich geringen Verlusten an Knochengewebe erlaubt. Die Augmentationsvorrichtung soll dauerhaft oder temporär in einen Patienten implantierbar sein.

### Bevorzugte Ausführungsformen der Erfindung

Ein Beitrag zur mindestens teilweisen Erfüllung mindestens einer der zuvor genannten Aufgaben wird durch die Merkmale des unabhängigen Anspruchs 1 geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Augmentationsvorrichtung umfassend einen ringförmigen Konus, der einen Kanal umgibt, welcher sich axial entlang einer Längsachse der Augmentationsvorrichtung von einem proximalen Konusende zu einem distalen Konusende erstreckt, wobei ein Außendurchmesser des Konus vom proximalen Konusende in Richtung des distalen Konusende abnimmt, insbesondere stetig abnimmt, wobei auf einer Mantelfläche des Konus mindestens zwei einer Außenkontur des Konus folgenden Metallplatten, insbesondere gebogene Metallplatten, angeordnet sind, wobei der Konus von den Metallplatten durch Verschieben des Konus in Richtung des proximalen Konusende reversibel lösbar ist.

In einer Ausführungsform der Augmentationsvorrichtungen sind die Metallplatten durch axial verlaufende Spalte zwischen den Metallplatten voneinander beanstandet. Diese Ausführungsform ist eine zweite Ausführungsform der Erfindung, welche vorzugsweise von der ersten Ausführungsform der Erfindung abhängt.

In einer Ausführungsform der Augmentationsvorrichtungen sind an der Metallfläche des Konus axial verlaufende Stege ausgebildet, welche in die Spalte zwischen den Metallplatten eingreifen. Diese Ausführungsform ist eine dritte Ausführungsform der Erfindung, welche vorzugsweise von der zweiten Ausführungsform der Erfindung abhängt.

In einer Ausführungsform der Augmentationsvorrichtung weisen die Stege mindestens ein Stegleitungsmittel auf, welche das proximale Konusende mit dem distalen Konusende und/oder das proximale Konusende mit einer Stegaußenfläche, insbesondere einer den Metallplatten zugewandten Stegaußenfläche, fluidleitend verbindet. Diese Ausführungsform ist eine vierte Ausführungsform der Erfindung, welche vorzugsweise von der dritten Ausführungsform der Erfindung abhängt.

In einer Ausführungsform der Augmentationsvorrichtung weist der Konus mindestens ein Konusleitungsmittel auf, welche das proximale Konusende und das distale Konusende fluidleitend verbindet. Diese Ausführungsform ist eine fünfte Ausführungsform der Erfindung, welche vorzugsweise von einer der vorhergehenden Ausführungsformen der Erfindung abhängt.

In einer Ausführungsform der Augmentationsvorrichtung weist der Konus am proximalen Konusende ein Schiebeelement auf, welches mit den Metallplatten an einem dem proximalen Konusende zugewandten proximalen Metallplattenende derart zusammenwirkt, dass eine Krafteinwirkung auf den Konus aus Richtung des proximalen Konusende über das Schiebeelement auf die Metallplatten übertragbar ist. Diese Ausführungsform ist eine sechste Ausführungsform der Erfindung, welche vorzugsweise von einer der vorhergehenden Ausführungsformen der Erfindung abhängt.

In einer Ausführungsform der Augmentationsvorrichtung sind die Metallplatten derart an der Mantelfläche des Konus angeordnet, dass zwischen den Metallplatten und der Mantelfläche eine reversibel formschlüssige Verbindung ausgebildet ist. Diese Ausführungsform ist eine siebte Ausführungsform der Erfindung, welche vorzugsweise von einer der vorhergehenden Ausführungsformen der Erfindung abhängt.

In einer Ausführungsform der Augmentationsvorrichtung ist eine Konuswanddicke des Konus größer oder gleich einer Metallplattenwanddicke der Metallplatten. Diese Ausführungsform ist eine achte Ausführungsform der Erfindung, welche vorzugsweise von einer der vorhergehenden Ausführungsformen der Erfindung abhängt.

In einer Ausführungsform der Augmentationsvorrichtung bedecken die Metallplatten mindestens 80 Flächen-%, bevorzugt 85 Flächen-%, weiter bevorzugt 90 Flächen-%, der Mantelfläche des Konus. Diese Ausführungsform ist eine neunte Ausführungsform der Erfindung, welche vorzugsweise von einer der vorhergehenden Ausführungsformen der Erfindung abhängt.

In einer Ausführungsform der Augmentationsvorrichtung ist die der Mantelfläche des Konus abgewandte Metallplattenaußenfläche aufgeraut, porös und/oder weist Sacklochbohrungen auf. Diese Ausführungsform ist eine zehnte Ausführungsform der Erfindung, welche vorzugsweise von einer der vorhergehenden Ausführungsformen der Erfindung abhängt.

In einer Ausführungsform der Augmentationsvorrichtung weisen die Sacklochbohrungen einen Durchmesser von kleiner 3 mm, bevorzugt von kleiner 2 mm, weiter bevorzugt von kleiner 1 mm, auf, wobei der Durchmesser vorzugsweise nicht kleiner ist als 0,5 mm. Diese Ausführungsform ist eine elfte Ausführungsform der Erfindung, welche vorzugsweise an der zehnten Ausführungsform der Erfindung abhängt.

In einer Ausführungsform der Augmentationsvorrichtung weist der Konus Durchführungen auf, welche den Kanal mit einem Zwischenraum zwischen dem Konus und den Metallplatten fluidleitend verbinden. Diese Ausführungsform ist eine zwölfte Ausführungsform der Erfindung, welche vorzugsweise von einer der vorhergehenden Ausführungsformen der Erfindung abhängt.

In einer Ausführungsform der Augmentationsvorrichtung ist an einem dem distalen Konusende zugewandten distalen Kanalende ein Abstreifelement angeordnet. Diese Ausführungsform ist eine dreizehnte Ausführungsform der Erfindung, welche vorzugsweise von einer der vorhergehenden Ausführungsformen der Erfindung abhängt.

In einer Ausführungsform der Augmentationsvorrichtungen umfassen die Metallplatten ein biokompatibles Metall, insbesondere bestehen die Metallplatten aus einem biokompatiblen Metall. Diese Ausführungsform ist eine vierzehnte Ausführungsform der Erfindung, welche vorzugsweise von einer der vorhergehenden Ausführungsformen der Erfindung abhängt.

In einer Ausführungsform der Augmentationsvorrichtung umfasst der Konus ein biokompatibles Polymer, insbesondere besteht der Konus aus einem biokompatiblen Polymer. Diese Ausführungsform ist eine fünfzehnte Ausführungsform der Erfindung, welche vorzugsweise von einer der vorhergehenden Ausführungsformen der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Die Begriffe "proximal" und "distal" dienen lediglich der Bezeichnung der räumlich entgegengesetzten Enden der Augmentationsvorrichtung, des Konus, oder anderer Struktureinheiten der Augmentationsvorrichtung und erlauben keinen Rückschluss auf die Orientierung der in einen menschlichen Körper implantieren Augmentationsvorrichtung. "Distal zu ..." und "proximal zu..." oder ähnliche Formulierungen drücken entsprechend lediglich die räumliche Anordnung zweier Struktureinheiten der Augmentationsvorrichtung und des Augmentationssystems zueinander aus.

### Ausführliche Beschreibung

Ein erster Gegenstand der Erfindung betrifft eine Augmentationsvorrichtung umfassend einen ringförmigen Konus, der einen Kanal umgibt, welcher sich axial entlang einer Längsachse der Augmentationsvorrichtung von einem proximalen Konusende zu einem distalen Konusende erstreckt, wobei ein Außendurchmesser des Konus vom proximalen Konusende in Richtung des distalen Konusende abnimmt, wobei auf einer Mantelfläche des Konus mindestens zwei, insbesondere zwei, drei, vier, fünf oder sechs, einer Außenkontur des Konus folgenden Metallplatten, insbesondere gebogene Metallplatten, angeordnet sind, wobei der Konus von den Metallplatten durch Verschieben des Konus in Richtung des proximalen Konusende reversibel lösbar ist.

Die Augmentationsvorrichtung weist einen ringförmigen Konus auf. Ein Konus ist ein kegelartiges, insbesondere kegelstumpfartig, geformtes Bauteil mit einem proximalen Konusende und einem dem proximalen Konusende axial, entlang der Längsachse der Augmentationsvorrichtung, gegenüberliegendem distalen Konusende. Der Konus weist einen Außendurchmesser auf, welcher vom proximalen Konusende in Richtung des distalen Konusende abnimmt. Der Konus weist somit am proximalen Konusende den größten Außendurchmesser und am distalen Konusende den kleinsten Außendurchmesser auf. Vorzugsweise verringert sich der Außendurchmesser vom proximalen Konusende in Richtung des distalen Konusende stetig.

Der Konus ist ringförmig, oder in anderen Worten rohrartig, ausgestaltet. Der ringförmige Konus umschließt somit einen Kanal, welcher sich axial durch den Konus vom proximalen Konusende bis zum distalen Konusende erstreckt. Der Kanal wird durch eine Konusinnenfläche ausgebildet. Der Begriff "ringförmig" umfasst in einer axialen Aufsicht auf den Konus Körper mit einem kreisförmigen, elliptischen, eckigen, beispielweise vier-, fünf- oder sechseckigen, und unregelmäßigen Umfang, wobei Körper mit elliptischem Umfang aufgrund einer besseren Implantierbarkeit in einen Patienten, insbesondere in einen Knochenkanal eines Patienten, bevorzugt sind.

Der Konus kann einteilig oder durch eine Vielzahl, also durch zwei oder mehr, miteinander verbundener, insbesondere reversibel verbundener, ringförmiger Konussegmente ausgebildet sein. Der Konus kann beispielsweise aus 2 bis 20 Konussegmenten, bevorzugt aus 4 bis 15, weiter bevorzugt aus 4 bis 10 Konussegmenten ausgebildet sein.

Jedes Konussegment bildet dabei einen ringförmigen Abschnitt des Konus und einen entsprechenden scheibenförmigen Abschnitt des Kanals des Konus ab. Dabei sind die Konussegmente so "übereinandergestapelt", dass diese den Konus ausbilden.

Die Konussegmente weisen einen Konussegmentaußendurchmesser auf, wobei sich die jeweiligen Konussegmentaußendurchmesser der einzelnen Konussegmente unterscheiden.

Das Konussegment mit dem größten Konussegmentaußendurchmesser formt das proximale Konusende aus, wobei sich der Konussegmentaußendurchmesser der sich in distaler Richtung anschließender Konussegmente immer weiter verringert, bis schließlich das Konussegment mit dem kleinsten Konussegmentaußendurchmesser das distale Konusende bildet.

Die Konussegmente weisen dabei Konussegmentaußendurchmesser in einem Bereich von 20 bis 60 mm, bevorzugt 25 bis 55 mm, weiter bevorzugt 30 bis 55 mm auf. Unter dem Konussegmentaußendurchmesser ist bei Konussegmenten, welche in einer axialen Aufsicht einen elliptischen Umfang aufweisen, die Länge der Hauptachse, also die längere der durch den Mittelpunkt verlaufenden Achsen gemeint.

Dies erlaubt eine gute Anpassung der Konusgröße an die anatomischen Gegebenheiten eines Patienten.

Die Konussegmente können unterschiedliche Bauhöhen umfassen. Beispielsweise besitzen die einzelnen Konussegmente eine Bauhöhe im Bereich von 1 mm bis 10 mm, bevorzugt im Bereich von 2 mm bis 8 mm. Dies erlaubt eine schnelle, effektive und gleichzeitig ausreichend kleinstufige Anpassung der axialen Erstreckung der Augmentationsvorrichtung.

Der Kanal dient der Aufnahme und Fixierung eines Schafts einer Prothese, insbesondere einer Gelenkendoprothese oder einer Revisionsgelenkendoprothese. Dafür kann der Schaft der entsprechenden Prothese in den Kanal, insbesondere von einem dem proximalen Konusende zugewandten proximalen Kanalende aus, eingebracht werden. Der Kanal weist einen Kanaldurchmesser auf, welcher von einem Konusinnendurchmesser bestimmt ist. In einer Ausgestaltungsform ist der Konusinnendurchmesser über die gesamte axiale Erstreckung des Kanals beziehungsweise des Konus konstant ausgestaltet, was zu einem über den gesamten Verlauf des Kanals konstanten Kanaldurchmesser führt. In einer weiteren, bevorzugten Ausgestaltungsform verringert sich der Konusinnendurchmesser, vorzugsweise gleichlaufend, zum Konusaußendurchmesser vom proximalen Konusende in Richtung des distalen Konusende. In dieser Ausgestaltungsform weist der Konus eine über die gesamte axiale Erstreckung des Konus im Wesentlichen gleich starke Konuswanddicke auf und gleichzeitig nimmt der Kanaldurchmesser vom proximalen Konusende in Richtung des distalen Konusende ab.

Die Konuswanddicke kann beispielsweise in einem Bereich von 2 mm bis 30 mm, bevorzugt 2 mm bis 15 mm, weiter bevorzugt 2 mm bis 10 mm, liegen.

Der Konus weist eine Mantelfläche auf. Die Mantelfläche ist der Konusinnenfläche gegenüberliegend angeordnet und stellt zumindest anteilig eine Konusaußenfläche dar. In einer Ausführungsform entspricht die Mantelfläche der Konusaußenfläche.

Auf der Mantelfläche des Konus sind mindestens zwei, also zwei oder mehr als zwei, Metallplatten, insbesondere gebogene Metallplatten, reversibel lösbar angeordnet. Die Metallplatten sind disjunkt voneinander anbringbar und lassen sich jeweils wiederholbar von der Mantelfläche lösen und wieder anbringen, beispielsweise durch Anlegen oder Andrücken.

Die Metallplatten sind derart am Konus angeordnet, dass die Mantelfläche von den Metallplatten zumindest teilweise ummantelt ist und die Metallplatten somit zumindest einen Teil einer im implantierten Zustand dem Knochen zugewandten Augmentationsvorrichtungsaußenfläche ausbilden. Vorzugsweise sind die Metallplatten überlappungsfrei an der Mantelfläche angeordnet.

Die Metallplatten sind derart geformt, dass sie einer Außenkontur des Konus folgend am Konus, insbesondere an der Mantelfläche des Konus, angeordnet sind. In einer Ausführungsform der Augmentationsvorrichtung liegen die Metallplatten formschlüssig an der Mantelfläche des Konus an. Die Mantelfläche des Konus ist dabei konvex ausgestaltet und eine Metallplatteninnenfläche der Metallplatten ist entsprechend der Kontur der Mantelfläche folgend konkav, also gebogen, ausgestaltet.

Vorzugsweise ist eine der Metallplatteninnenfläche gegenüberliegend angeordnete Metallplattenaußenfläche der Metallplatten konvex ausgeformt. Weiter bevorzugt weisen die Metallplatten eine gleichmäßige Metallplattenwanddicke auf.

Um die Augmentationsvorrichtung in einen Knochen, insbesondere einen Knochenkanal, zu Implantieren, wird diese mit an der Mantelfläche angeordneten Metallplatten mit dem distalen Konusende voran das vorbereitete Implantatlager des Knochens geschoben. Dabei werden die Metallplatten über eine Krafteinwirkung auf den Konus aus Richtung des proximalen Konusende gegen das Knochengewebe gepresst und so die Augmentationsvorrichtung im Implantatlager fixiert. Der Konus stellt dabei ein Widerlager für die Metallplatten dar.

Nach der Fixierung der Augmentationsvorrichtung im Implantatlager kann eine Prothese, insbesondere ein Schaft einer Prothese, aus Richtung des proximalen Konusende in den Kanal der Augmentationsvorrichtung eingeführt und dort, beispielsweise mittels eines Knochenzements, verankert werden.

Sollte eine Explantation des Implantats nötig sein, kann die zu entfernende Prothese, insbesondere Revisionsgelenkendoprothese, axial in Richtung des proximalen Konusende aus der Augmentationsvorrichtung herausgezogen werden. Vorzugsweise ist der Konus im Wesentlichen nicht mit Knochengewebe über Aufwachsen oder Einwachsen verbunden, so dass in Abhängigkeit der Verankerung von Prothese zu Konus, beispielsweise bei fester Verankerung mittels eines Knochenzements, beim Herausziehen der Prothese gleichzeitig der Konus aus dem Knochen entfernt wird. Der Konus lässt sich axial in Richtung des proximalen Konusende lösbar gegen die Metallplatten verschiebbar, um die Metallplatten und den Konus reversibel zu trennen. Dies stellt eine schnelle, einfache und sichere Explantation der Prothese dar.

Die Metallplatten, welche vorzugsweise mit Knochengewebe verwachsen sind, verbleiben nach dem Entfernen des Konus am Knochen. Da mit Entfernung des Konus das Widerlager entfernt wurde, welches die Metallplatten gegen den Knochen drückt, können die Metallplatten radial in Richtung der Längsachse des Knochens gezogen und so vom Knochen gelöst werden. Dies führt zu weniger Verlust an Knochengewebe als eine axiale, in Längsrichtung des Knochens ausgeführte, Ziehbewegung und erleichtert die Explantation der Augmentationsvorrichtung. Zudem verringert sich dadurch die Gefahr eines hohen Verlusts an Knochengewebe.

Die Metallplatten dienen einem verbesserten Aufwachsen, insbesondere Einwachsen, von Knochengewebe auf oder in die Augmentationsvorrichtung im implantierten Zustand.

Ein Aufwachsen oder Einwachsen von Knochengewebe auf oder in den Konus wird vorzugsweise unterbunden. Dies wird beispielsweise dadurch erreicht, dass der Konus im implantierten Zustand im Wesentlichen nicht mit Knochengewebe in Berührung kommt, insbesondere da der Konus im Wesentlichen manschettenartig von den Metallplatten ummantelt ist. In einer weiteren Ausführungsform ist der Konus nicht aus Metall ausgebildet, so dass ein Aufwachsen oder Einwachsen von Knochengewebe auf oder in den Konus im Wesentlichen unterbleibt.

Um eine Explantation der Augmentationsvorrichtung weiter zu erleichtern und eine die Gefahr eines hohen Verlustes an Knochengewebe im Zuge der Explantation weiter zu minimieren, sind in einer Ausführungsform der Augmentationsvorrichtung die Metallplatten derart an der Mantelfläche des Konus angeordnet, dass diese durch axial, in Längsrichtung der Augmentationsvorrichtung, verlaufende Spalte voneinander beabstandet sind. Die Anzahl der Spalte hängt von der Anzahl der Metallplatten ab. Beispielsweise sind zwei Metallplatten derart an der Mantelfläche des Konus angeordnet, dass diese durch zwei Spalte voneinander beabstandet sind.

In einer Ausführungsform verlaufen zumindest einer der Spalte und die Längsachse der Vorrichtung in einer gemeinsamen Ebene. Vorzugsweise verlaufen die Spalte der Augmentationsvorrichtung mit angeordneten Metallplatten entlang des kürzesten Weges vom proximalen Konusende zum distalen Konusende

Die Spalte zwischen den Metallplatten bleiben auch im implantierten Zustand der Augmentationsvorrichtung erhalten, so dass die Metallplatten bei einer Explantation nach erfolgtem Lösen des Konus von den Metallplatten mittels axialen Verschiebens des Konus in Richtung des proximalen Konusende leichter radial in Richtung der Längsachse des Knochens ziehbar und somit vom Knochen entfernbar sind. Durch die axial verlaufenden Spalte kann jede der Metallplatten durch eine radiale Zugbewegung in Richtung der Längsachse des Knochens entfernt werden, ohne von einer oder mehreren weiteren, noch mit dem Knochengewebe verbundenen Metallplatten, sterisch behindert zu werden.

Die Spalte können unterschiedlich groß ausgestaltet sein, um die Metallplatten auf der Mantelfläche des Konus zu beabstanden. Um eine erleichterte Explantation zu gewährleisten, ist es bevorzugt, dass zumindest einer der Spalte, bevorzugt alle Spalte, eine Spaltbreite in einem Bereich von 1 mm bis 5 mm, bevorzugt in einem Bereich von 2 mm bis 5 mm, weiter bevorzugt in einem Bereich von 3 mm bis 4 mm aufweisen. Eine geringere Spaltbreite würde weniger zu einer erleichterten Explantation der Augmentationsvorrichtung beitragen, wohingegen eine größere Spaltbreite zu kleineren Metallplatten führen, so dass ein Aufwachsen oder Einwachsen von Knochengewebe auf oder in die Augmentationsvorrichtung vermindert ist.

Eine Ausführungsform der Augmentationsvorrichtung ist dadurch gekennzeichnet, dass an der der Mantelfläche des Konus axial verlaufende Stege ausgebildet sind, welche in die Spalte, insbesondere die axial verlaufenden Spalte, zwischen den Metallplatten eingreifen, so dass die Stege die Spalte zumindest teilweise, bevorzugt im Wesentlichen vollständig, ausfüllen. Vorzugsweise verlaufen die Stege über die gesamte axiale Erstreckung des Konus. In einer Ausgestaltungsform sind der Konus und die Stege mehrteilig ausgestaltet, wobei der Konus und die Stege beispielsweise über eine Klebeverbindung oder einen Kraftschluss miteinander verbunden sind. In einer weiteren, bevorzugten Ausgestaltungsform sind der Konus und die Stege einteilig ausgestaltet. Die Stege erleichtern das Anordnen der Metallplatten an der Mantelfläche, so dass jede Metallplatte an der ihr zugedachten Position an der Mantelfläche anbringbar ist. Zudem verhindern die Stege ein Verschieben der Metallplatten auf der Mantelfläche und insbesondere ein Aneinanderreiben der Metallplatten, welches insbesondere im implantierten Zustand der Augmentationsvorrichtung zu ungewollten Metallabrieb führen könnte.

Weiterhin verhindern die Stege, dass die Spalte zwischen den Metallplatten im implantierten Zustand der Augmentationsvorrichtung mit Gewebe, insbesondere Knochengewebe und Bindegewebe, zuwachsen, so dass bei einer Explantation der Augmentationsvorrichtung nach erfolgtem Entfernen des Konus die verbundenen Metallplatten leichter durch radialen Zug in Richtung der Längsachse des Knochens vom Knochengewebe lösbar sind.

Die Stege können massiv oder strukturiert, beispielsweise mit einer Wabenstruktur, ausgeformt sein.

Eine Ausführungsform der Augmentationsvorrichtung ist dadurch gekennzeichnet, dass die Stege mindestens ein fluidleitendes Stegleitungsmittel aufweisen, welche das proximale Konusende mit dem distalen Konusende, das proximale Konusende mit einer Stegaußenfläche oder das proximale Konusende mit dem distalen Konusende sowie das proximale Konusende und die Stegaußenfläche fluidleitend verbindet. Durch das mindestens eine Stegleitungsmittel können pharmazeutische Fluide, wie beispielsweise wässrige Antibiotikalösungen, vom proximalen Konusende, welches nach erfolgter Implantation der Augmentationsvorrichtung in das entsprechende Implantatlager gut für den Chirurgen erreichbar ist, in Richtung des distalen Konusende geleitet werden. Im Falle eines Stegleitungsmittels, welches das proximale Konusende mit dem distalen Konusende fluidleitend verbindet, können pharmazeutische Fluide zumindest in den distalen Bereich der Augmentationsvorrichtung gefördert werden. Im Falle eines Stegleitungsmittels, welches das proximale Konusende mit einer Stegaußenfläche fluidleitend verbindet, können pharmazeutische Fluide zumindest in den Bereich der Mantelfläche des Konus, und damit in den Bereich des Umfangs der Augmentationsvorrichtung, gefördert werden. In einer Ausgestaltungsform mündet das Stegleitungsmittel an einer dem Kanal abgewandten Stegaußenfläche. In einer weiteren, bevorzugten Ausgestaltungsform mündet das Stegleitungsmittel zumindest an einer der Metallplatten zugewandten Stegaußenfläche. Dabei kann das Steigleitungsmittel an einer oder mehreren Stellen an der den Metallplatten zugewandten Stegaußenfläche münden. Mündet das Stegleitungsmittel an einer der Metallplatte zugewandten Stegaußenfläche, verringert sich die Gefahr eines Einwachsens von Gewebe, insbesondere Knochengewebe oder Bindegewebe, in das Stegleitungsmittel, da diese durch die Metallplatte teilweise abgeschirmt ist und das Einwachsen des Gewebes so erschwert. Mündet das Stegleitungsmittel an einer der Metallplatte zugewandten Stegaußenfläche, ist es bevorzugt, dass die Metallplatte in der Metallplattenaußenfläche und/oder Metallplatteninnenfläche zumindest eine Nut, insbesondere eine radial und/oder axial über die gesamte Erstreckung der Metallplatte verlaufende Nut, aufweist, welche fluidleitend mit dem Stegleitungsmittel verbunden ist und für eine großflächigere Verteilung des pharmazeutischen Fluids an der Augmentationsvorrichtungsaußenfläche sorgt. Vorzugsweise ist jede Mündung des Stegleitungsmittel dabei mit einer Nut fluidleitend verbunden.

In einer Ausgestaltungsform verbindet ein Stegleitungsmittel sowohl das distale Konusende als auch eine Stegaußenfläche des Stegs fluidleitend mit dem proximalen Konusende.

Bevorzugt weisen alle Stege der Augmentationsvorrichtung zumindest ein Stegleitungsmittel auf.

Unter einem Stegleitungsmittel wird jede Art von Struktur verstanden, welche zumindest abschnittweise innerhalb des Stegs verläuft und welche das Fördern eines Fluids, wie einer Flüssigkeit oder eines Gases, von einem Ende des Stegleitungsmittel zu einem weiteren Ende des Stegleitungsmittels erlaubt. Das Stegleitungsmittel kann beispielsweise eine Bohrung, ein Schlauch oder ein Tunnel sein.

Eine Ausführungsform der Augmentationsvorrichtung ist dadurch gekennzeichnet, dass der Konus mindestens ein Konusleitungsmittel aufweist, welche das proximale Konusende und das distale Konusende fluidleitend miteinander verbindet. Durch das mindestens eine Konusleitungsmittel können pharmazeutische Fluide, wie beispielsweise wässrige AntibiotikaLösungen, vom proximalen Konusende, welches nach erfolgter Implantation der Augmentationsvorrichtung in das entsprechende Implantatlager gut für den Chirurgen erreichbar ist, an das distale Konusende gefördert werden.

Unter einem Konusleitungsmittel wird jede Art von Struktur verstanden, welche zumindest abschnittweise innerhalb des Konus verläuft und welche das Fördern eines Fluids, wie einer Flüssigkeit oder eines Gases, von einem proximalen Ende des Konusleitungsmittel zu einem distalen Ende des Konusleitungsmittels erlaubt. Das Konusleitungsmittel kann beispielsweise eine Bohrung, ein Schlauch oder ein Tunnel sein.

Eine Ausführungsform der Augmentationsvorrichtung ist dadurch gekennzeichnet, dass der Konus am proximalen Konusende ein Schiebeelement aufweist, welches mit den Metallplatten an einem dem proximalen Konusende zugewandten proximalen Metallplattenende derart zusammenwirkt, dass eine Krafteinwirkung auf den Konus aus Richtung des proximalen Konusende mittels des Schiebeelements auf die Metallplatten übertragbar ist. Das Schiebeelement erstreckt sich zumindest anteilig radial über oder aus der Mantelfläche des Konus heraus und ist proximal zum proximalen Metallplattenende angeordnet. Vorzugsweise ist das Schiebeelement als radial umlaufende Wulst am proximalen Konusende ausgeformt. Erfolgt eine Krafteinwirkung aus Richtung des proximalen Konusende, wirkt diese in Richtung des distalen Konusende wirkende Kraft auf das Schiebeelement, welche diese über seine distale, der Metallplatte zugewandte, Schiebeelementfläche auf das proximale, dem Schiebeelement zugewandte, proximale Metallplattenende übertragt und so die Metallplatte zusammen mit dem Konus in Richtung des distalen Konusende verschiebt. Das Schiebeelement sorgt dadurch für eine verbesserte Krafteinwirkung auf die Metallplatten und verringert das ungewollte Abrutschen der Metallplatten von der Mantelfläche im Zuge eines Einbringens der Augmentationsvorrichtung in ein Implantatlager.

In einer Ausführungsform der Augmentationsvorrichtung sind die Metallplatteninnenfläche und die Mantelfläche glatt ausgestaltet, so dass der Konus bereits durch eine möglichst geringe Krafteinwirkung auf den Konus in Richtung des proximalen Konusende reversibel von den Metallplatten lösbar ist.

Eine Ausführungsform der Augmentationsvorrichtung ist dadurch gekennzeichnet, dass die Metallplatten derart an der Mantelfläche des Konus angeordnet sind, dass zwischen den Metallplatten und der Mantelfläche eine reversibel formschlüssige Verbindung ausgebildet ist. Die reversibel formschlüssige Verbindung dient dazu, ein unabsichtliches Lösen der Metallplatten von der Mantelfläche des Konus, beispielsweise beim Transport oder der Implantation der Augmentationsvorrichtung, unterbunden wird. Die formschlüssige Verbindung ermöglicht weiterhin ein Lösen von Konus und Metallplatten durch axialen Zug des Konus in Richtung des proximalen Konusende. In einer Ausgestaltungsform ist die formschlüssige Verbindung zwischen den Metallplatten und dem Konus, insbesondere der Mantelfläche des Konus, als eine Nut-Feder-Verbindung ausgebildet. In einer weiteren Ausgestaltungsform ist die formschlüssige Verbindung zwischen den Metallplatten und dem Konus, insbesondere der Mantelfläche des Konus, als eine noppenartige oder Klemmbaustein-artige Verbindung ausgebildet.

Die Metallplatten und der Konus können unterschiedlich große Wanddicken aufweisen.

Eine Ausführungsform der Augmentationsvorrichtung ist dadurch gekennzeichnet, dass eine Konuswanddicke des Konus größer oder gleich einer Metallplattenwanddick der Metallplatten ist. So ist gewährleistet, dass der Konus als möglichst stabiles Widerlager für die Metallplatten im Zuge der Implantation und im implantierten Zustand der Augmentationsvorrichtung dienen kann. Zudem wird so eine pressfit-Verankerung der Augmentationsvorrichtung im Implantatlager erleichtert.

Die Konuswanddicke befindet sich vorzugsweise in einem Bereich von 2 mm bis 30 mm, bevorzugt in einem Bereich von 2 mm bis 15 mm, weiter bevorzugt in einem Bereich von 2 mm bis 10 mm. Die Metallplattenwanddick befindet sich vorzugsweise in einem Bereich von 1,5 mm bis 15 mm, bevorzugt in einem Bereich von 1,5 mm bis 10 mm, weiter bevorzugt in einem Bereich von 1,5 mm bis 5 mm.

Die Metallplatten können unterschiedliche große Flächenbereiche der Mantelfläche des Konus bedecken.

Um ein gutes Anwachsen oder Einwachsen von Knochengewebe auf oder in die Augmentationsvorrichtung, insbesondere die Metallplatten, und so eine stabile Verankerung der Augmentationsvorrichtung in einem Implantatlager zu ermöglichen, ist eine Ausführungsform der Augmentationsvorrichtung dadurch gekennzeichnet, dass die Metallplatten mindestens 80 Flächen-%, bevorzugt mindestens 90 Flächen-%, weiter bevorzugt mindestens 95 Flächen-% der Mantelfläche, bezogen auf die Gesamtfläche der Mantelfläche, bedecken.

Eine Ausführungsform der Augmentationsvorrichtung ist dadurch gekennzeichnet, dass die der Mantelfläche des Konus abgewandte Metallplattenaußenfläche aufgeraut ist, porös, insbesondere offenporös, ist und/oder Sacklochbohrungen aufweist. Die derart strukturierte Metallplattenaußenfläche erleichtert ein Aufwachsen oder Einwachsen von Knochengewebe und verbessert so die Verankerung der Augmentationsvorrichtung in einem Implantatlager. Vorzugsweise verlaufen die Sacklochbohrungen im Wesentlichen radial, insbesondere senkrecht, zur Längsachse der Augmentationsvorrichtung. Die Sacklochbohrungen sind vorzugsweise nicht untereinander verbunden, so dass keine Hinterschnitte an den Sacklochbohrungen ausgebildet werden, welche ein Explantieren der Metallplatten aus einem Implantatlager erschweren würden. Die aufgeraute Struktur der Metallplattenaußenfläche kann beispielsweise durch Sandstrahlen, insbesondere durch grobes Sandstrahlen, erfolgen.

Um ein verbessertes Einwachsen von Knochengewebe zu ermöglich, ist eine Ausführungsform der Augmentationsvorrichtung dadurch gekennzeichnet, dass die Sachlochbohrungen einen Durchmesser von kleiner 3 mm, bevorzugt von kleiner 2 mm, weiter bevorzugt von kleiner 1 mm aufweisen, wobei der Durchmesser vorzugsweise nicht kleiner als 0,5 mm ist. Dieser Bereich ermöglicht ein gutes Einwachsen von Knochengewebe in die Metallplatten und ist leicht zu fertigen.

Zwischen den Metallplatten und dem Konus, insbesondere zwischen den Metallplatteninnenflächen und der Mantelfläche des Konus, kann ein Zwischenraum ausgebildet sein. Der Zwischenraum kann beispielsweise gewollt herbeigeführt werden, beispielsweise durch Einkerbungen in den Metallplatten und/oder dem Konus, produktionstechnisch bedingt sein und/oder aufgrund eines Verrutschens der Metallplatten auf der Mantelfläche im Zuge einer Implantation der Augmentationsvorrichtung in ein Implantatlager. Um Materialabrieb zu verringern oder gänzlich zu vermeiden, ist eine Ausführungsform der Augmentationsvorrichtung dadurch gekennzeichnet, dass der Konus zumindest eine Durchführung aufweist, welche den Kanal mit dem Zwischenraum zwischen dem Konus und mindestens einer der Metallplatten fluidleitend verbinden. Dies erlaubt ein Fördern eines Knochenzementteigs, welcher beispielsweise zur Verankerung einer Prothese in den Kanal appliziert wird, durch die mindestens eine Durchführung in den Zwischenraum. Der Zwischenraum kann über die zumindest eine Durchführung mit dem Knochenzementteig gefüllt, welcher dort zu einem Knochenzement aushärtet. Der Knochenzement im Zwischenraum zwischen dem Konus und der mindestens einen Metallplatte unterbindet wirksam Mikrobewegungen und verringert oder unterbindet dadurch Materialabrieb.

Eine Ausführungsform der Augmentationsvorrichtung ist dadurch gekennzeichnet, dass an einem dem distalen Konusende zugewandten distalen Kanalende ein Abstreifelement angeordnet ist. Das Abstreifelement dient dazu, dass ein Knochenzementteig, welcher in den Kanal, insbesondere in das proximalen Kanalende, eingefüllt wird weitgehend im Kanal verbleibt und nicht aus dem distalen Kanalende ausgebracht wird. Gleichzeitig ermöglicht das Abstreifelement ein Durchführen eines Prothesenschafts durch den Kanal zur Verankerung einer Prothese in der Augmentationsvorrichtung. Dazu kann der Prothesenschaft aus Richtung des proximalen Kanalendes durch den Kanal und durch das Abstreifelement geschoben werden, so dass der Prothesenschaft zumindest abschnittsweise aus dem distalen Kanalende herausragt. Das Abstreifelement streift beim Schieben des Prothesenschafts durch das Abstreifelement weitgehend am Prothesenschaft anhaftenden Knochenzementteig ab, so dass dieser innerhalb des Kanals verbleibt und ein weitgehend von Knochenzementteig befreiter Prothesenschaft aus dem distalen Kanalende austritt.

Das Abstreifelement kann unterschiedlich ausgeformt sein, um die gewünschte Wirkung zu erzielen. Beispielsweise kann das Abstreifelement als eine Platte, insbesondere eine elastisch verformbare Platte, beispielsweise aus einem Polymer, ausgebildet sein, welche eine Aussparung im Wesentlichen in Form einer Querschnittsgeometrie eines Prothesenschaftes aufweist. Die Platte verschließt dabei das distalen Kanalende fluidleitend für einen Knochenzementteig mit Ausnahme der Aussparung, durch welche der Prothesenschaft axial durchschiebbar. Beim Durchschieben durch die Aussparung streift das im Wesentlichen am Prothesenschaft anliegend Abstreifelement am Prothesenschaft anhaftenden Knochenzementteig ab.

In einer weiteren Ausgestaltungsform ist das Abstreifelement ebenfalls als eine Platte, insbesondere eine elastisch verformbare Platte, beispielsweise aus einem Polymer, ausgebildet, wobei die Platte anstelle der Aussparung zumindest zwei, beispielsweise zwei, drei oder vier, Einschnitte aufweist, welche sich in einem Punkt kreuzen. Die Einschnitte bilden Plattenabschnitte aus, welche im Ruhezustand das distale Kanalende im Wesentlichen fluidleitend für einen Knochenzementteig verschließen. Bei hoher Druckbeaufschlagung, insbesondere durch einen durch den Kanal vom proximalen Kanalende geschobenen Prothesenschaft, biegen sich die Plattenabschnitte aus dem distalen Kanalende heraus, wodurch ein Prothesenschaft aus dem distalen Kanalende herausgeschoben werden kann. Dabei streifen die Plattenabschnitte am Prothesenschaft anhaftenden Knochenzementteig ab, so dass dieser im Wesentlichen frei von Knochenzementteig ist.

Eine Ausführungsform der Augmentationsvorrichtung ist dadurch gekennzeichnet, dass die Metallplatten ein biokompatibles Metall umfassen, bevorzugt dass die Metallplatten aus einem biokompatiblen Metall bestehen. Beispiele für biokompatible Metalle sind Titan, Titanlegierungen, Tantal, Tantallegierungen und Edelstähle, wobei Titan und Titanlegierungen bevorzugt sind.

Der Konus kann unterschiedliche Materialien umfassen oder aus unterschiedlichen Materialen bestehen.

Um ein Aufwachsen oder Einwachsen von Knochengewebe auf oder in den Konus weitestgehend zu unterbinden oder zumindest zu erschweren, ist eine Ausführungsform der Augmentationsvorrichtung dadurch gekennzeichnet, dass der Konus ein biokompatibles Polymer umfasst, bevorzugt dass der Konus aus einem biokompatiblen Polymer besteht. Beispiele für biokompatible Polymere sind Polyetherketon, Polyamid, Polyimid, Polysulfon und Knochenzement, insbesondere PMMA-Knochenzement, wobei Knochenzement, insbesondere PMMA-Knochenzement, bevorzugt ist. Ein Konus aus Knochenzement, insbesondere PMMA-Knochenzement, verbessert die Fixierung einer Prothese innerhalb der Augmentationsvorrichtung mittels eines Knochenzements, insbesondere PMMA-Knochenzements.

Das biokompatible Polymer, insbesondere der PMMA-Knochenzement, kann ein oder mehrere pharmazeutische Wirkstoffe, insbesondere ein oder mehrere Antibiotika, enthalten. Bevorzugte Antibiotika sind dabei Gentamicin und Tobramycin. Die im Konus enthaltenen pharmazeutischen Wirkstoffe, insbesondere Antibiotika, können nach erfolgter Implantation der Augmentation in ein Implantatlager zumindest über das proximale Konusende und das distale Konusende freigesetzt werden.

Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochengewebe zu erstellen und/oder Wirbelkörper zu stabilisieren. Durch Aushärtung wird aus einem Knochenzementteig ein Knochenzement. Bevorzugt handelt es sich bei diesen Knochenzementen um Polymethylmethacrylat-Knochenzemente (PMMA-Knochenzemente) oder um anorganische Knochenzemente. PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einer Pulverkomponente umfassend ein Knochenzementpulver als erster Ausgangskomponente und einer Flüssigkeitskomponente umfassend eine Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer

Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin. Unter einem anorganischen Knochenzement wird ein Knochenzement auf Basis von Calciumphosphaten und Calciumsulfat-Dihydrat verstanden. Als Pulverkomponente kommen dabei Pulver von Calciumphosphaten und/oder Calciumsulfat-Dihydrat zum Einsatz, welche durch eine Flüssigkeitskomponente umfassend eine wässrige Lösung verschiedener Salze aushärtbar ist. Es wurde eine Vielzahl an anorganischen Knochenzementen beschrieben, von denen exemplarisch folgende genannt sind: EP 1 592 463 B1, EP 2 271 585 B1 und EP 2 988 789 B1.

### Figuren

Die Erfindung wird im Folgenden durch Figuren weiter beispielhaft illustriert. Die Erfindung ist nicht auf die Figuren beschränkt.

Es zeigen
- Fig. 1: eine schematische perspektivische Seitenansicht einer Augmentationsvorrichtung in einem getrennten Zustand,
- Fig. 2: die Augmentationsvorrichtung aus Figur 1 in einer perspektivischen Seitenansicht in einem zusammengesetzten Zustand,
- Fig. 3: eine schematische perspektivische Seitenansicht einer weiteren Augmentationsvorrichtung,
- Fig. 4: eine schematische Explosionsdarstellung einer weiteren Augmentationsvorrichtung,
- Fig. 5: eine schematische perspektivische Schnittdarstellung einer weiteren Augmentationsvorrichtung
- Fig. 6: eine schematische perspektivische Schnittdarstellung einer weiteren Augmentationsvorrichtung
- Fig. 7: eine schematische perspektivische Seitenansicht einer weiteren Augmentationsvorrichtung in einem getrennten Zustand,
- Fig. 8: eine schematische perspektivische Seitenansicht einer weiteren Augmentationsvorrichtung.

### Beschreibung der Figuren

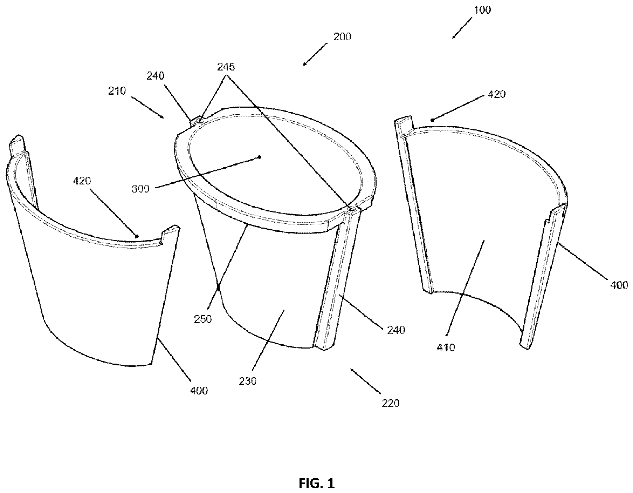

**Figur 1** zeigt eine schematische Seitenansicht einer Augmentationsvorrichtung 100 in einem getrennten Zustand. Die Augmentationsvorrichtung 100 umfasst einen ringförmigen Konus 200, durch welchen sich axial von einem proximalen Konusende 210 bis zu einem distalen Konusende 220 ein fluidleitender Kanal 300 erstreckt. Der Konus 200 weist einen Außendurchmesser auf, welcher vom proximalen Konusende 210 in Richtung des distalen Konusende 220 abnimmt. Der Konus 200 weist einen ellipsenförmigen Querschnitt auf.

Der Konus 200 weist eine Mantelfläche 230 und zwei an der Mantelfläche 230 sich gegenüberliegende, axial verlaufende Stege 240 (lediglich einer der Stege 240 ist vollständig sichtbar) auf. Der Konus 200 und die Stege 240 sind einteilig ausgestaltet. Axial durch die Stege 240 verläuft jeweils ein kanalartiges Stegleitungsmittel 245, welche das proximale Konusende 210 fluidleitend mit dem distalen Konusende 220 verbindet. Die Stegleitungsmittel 245 erlauben ein Applizieren eines pharmazeutischen Fluids, beispielsweise einer wässrigen Antibiotikalösung, über das im implantierten Zustand der Augmentationsvorrichtung 100 für den Chirurgen leicht zugängliche proximale Konusende 210 an das im implantierten Zustand der Augmentationsvorrichtung 100 für den Chirurgen schwer zugängliche distale Konusende 220.

Die Augmentationsvorrichtung 100 umfasst neben dem Konus 200 zwei gebogenen Metallplatten 400, wobei die Metallplatten derartig geformt sind, dass diese einer Außenkontur des Konus folgenden an der Mantelfläche 230 anliegend angeordnet werden können. In Figur 1 sind der Konus 200 und die Metallplatten 400 der Augmentationsvorrichtung 100 getrennt voneinander dargestellt.

Die Metallplatten 400 sind insbesondere an einer dem Konus 200 zugewandten Metallplatteninnenfläche 410 so geformt, dass die Metallplatten 400, insbesondere die Metallplatteninnenfläche 410, der Außenkontur des Konus 200 derart folgen, dass die Metallplatten 400 an der Mantelfläche 230 anliegend angeordnet werden können.

Der Konus 200 umfasst weiterhin am proximalen Konusende 210 ein radial um den Konus 200, insbesondere das proximale Konusende 210, laufendes Schiebeelement 250. Die Metallplatten 400 weisen jeweils eine Metallplattenaussparung 420 auf, in welche das Schiebeelement 250 eingreifen kann, wenn die Metallplatten 400 an der Mantelfläche angeordnet sind. Dies erleichtert das Anordnen und verbessert die Haftung der Metallplatten 400 an der Mantelfläche 230.

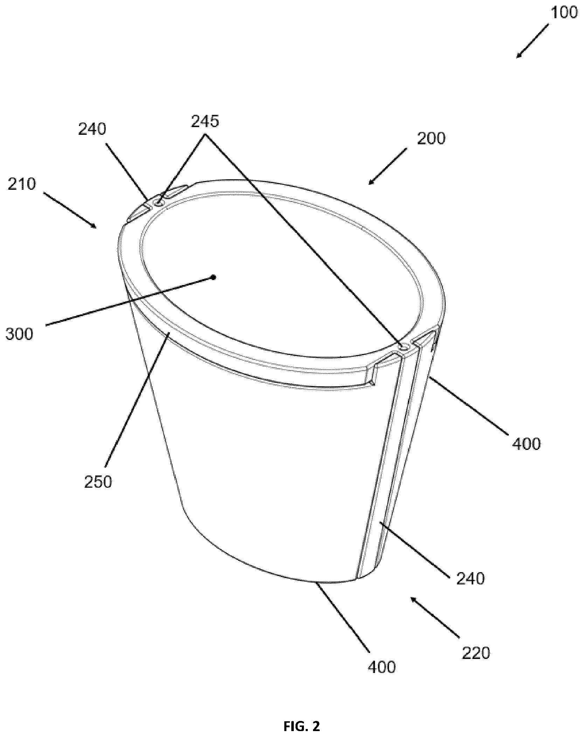

**Figur 2** zeigt die Augmentationsvorrichtung 100 aus Figur 1 in einer perspektivischen Seitenansicht in einem zusammengesetzten Zustand. Die Metallplatten 400 sind am Konus 200 angeordnet und liegen an der Mantelfläche 230 (vgl. Figur 1) an, so dass die Metallplatteninnenflächen 410 (vgl. Figur 1) im Wesentlichen vollständig an der Mantelfläche 230 (vgl. Figur 1) anliegen. Im zusammengesetzten Zustand greift das Schiebeelement 250 in die Metallplattenaussparungen 420 (vgl. Figur 1) ein, so dass eine axiale Krafteinwirkung auf den Konus 200 aus Richtung des proximalen Konusende 210 in Richtung des distalen Konusende 220 über das Schiebelement 250 auf die Metallplatten 400 übertragen wird. Dies erleichtert die Implantationsfähigkeit der Augmentationsvorrichtung 100 in einen Patienten und sorgt dafür, dass die Metallplatten 400 während der Implantation nicht auf der Mantelfläche 230 (vgl. Figur 1) in Richtung des proximalen Konusende 210 verrutschen. Der Metallplatten 400 liegen im zusammengesetzten Zustand sowohl an der Mantelfläche 230 (vgl. Figur 1), als auch an den Stegen 240 an. Durch die Stege 240 sind die Metallplatten 400 voneinander beabstandet. Zwischen den Metallplatten 400 sind Spalte (nicht gezeigt) ausgebildet, welche von den Stegen 240 im Wesentlichen vollständig ausgefüllt sind. Die Stege 240 verhindern ein unbeabsichtigtes Verrutschen der Metallplatten 400 am Konus 200.

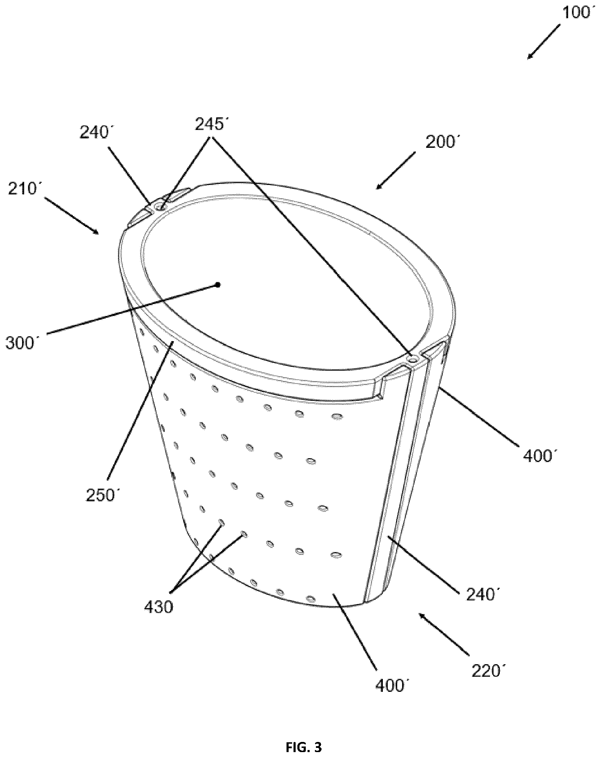

**Figur 3** zeigt eine perspektivische Seitenansicht einer weiteren Ausführungsform einer Augmentationsvorrichtung 100' in einem zusammengesetzten Zustand. Die Ausführungsform der Augmentationsvorrichtung 100' stimmt weitgehend mit der vorstehend beschriebenen und in den Figuren 1 und 2 dargestellten Ausführungsform überein, so dass zur Vermeidung von Wiederholungen auf die vorstehende Beschreibung verwiesen wird. Abwandlungen einer gegenüber der in den Figuren 1 und 2 gezeigten Ausführungsform weisen dasselbe Bezugszeichen mit einem Apostroph auf.

Die Augmentationsvorrichtung 100' unterscheidet sich von der Augmentationsvorrichtung 100 aus den Figuren 1 und 2 durch in den Metallplatten 400' vorhandenen Sacklochbohrungen 430 (lediglich exemplarisch beziffert und lediglich auf der dem Betrachter der Zeichnung zugewandten Metallplatte 400' sichtbar). Die Sacklochbohrungen 430 verbessern ein Auf- und Einwachsen von Knochengewebe auf und in die Metallplatten 400'.

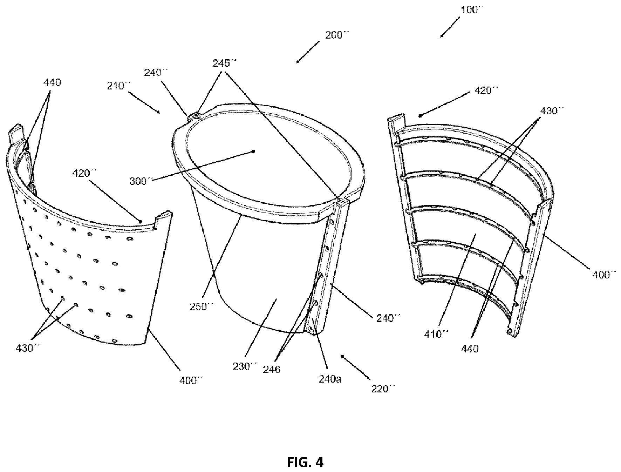

**Figur 4** zeigt eine perspektivische Seitenansicht einer weiteren Ausführungsform einer Augmentationsvorrichtung 100" in einem getrennten Zustand. Die Ausführungsform der Augmentationsvorrichtung 100" stimmt weitgehend mit der vorstehend beschriebenen und in den Figuren 1 bis 3 dargestellten Ausführungsformen überein, so dass zur Vermeidung von Wiederholungen auf die vorstehende Beschreibung verwiesen wird. Abwandlungen einer gegenüber den in den Figuren 1 bis 3 gezeigten Ausführungsform weisen dasselbe Bezugszeichen mit zwei Apostrophen auf.

Die Ausführungsform der Augmentationsvorrichtung 100" unterscheidet sich von der Ausführungsform der Augmentationsvorrichtung 100' aus Figur 3 dadurch, dass die axial durch die Stege 240" verlaufenden kanalartigen Stegleitungsmittel 245" weitere Stegleitungsmittelöffnungen 246 (lediglich exemplarisch beziffert) an den den Metallplatten 400" im zusammengesetzten Zustand der Augmentationsvorrichtung 10" zugewandten Stegaußenflächen 240a aufweisen. Dadurch kann ein pharmazeutisches Fluid, beispielsweise eine wässrige Antibiotikalösung, nicht nur an das distalen Konusende 220", sondern zusätzlich auch über die axiale Erstreckung der Augmentationsvorrichtung 100" appliziert werden. Um bei einer Applikation eines in die Stegleitungsmittel 245" am proximalen Konusende 210" applizierten pharmazeutischen Fluids eine möglichst gleichmäßige Verteilung über den gesamten Umfang der Augmentationsvorrichtung 100" zu erreichen, sind die weiteren Stegleitungsmittelöffnungen 246 im zusammengesetzten Zustand der Augmentationsvorrichtung 100" fluidleitend mit an den Metallplatteninnenflächen 410" verlaufenden inneren Nuten 440 verbunden. Die inneren Nuten 440 sind wiederum fluidleitend mit den Sacklochbohrungen 430" verbunden, um eine möglichst gleichmäßige Verteilung eines pharmazeutischen Fluid über den Umfang der Augmentationsvorrichtung 100" zu ermöglichen.

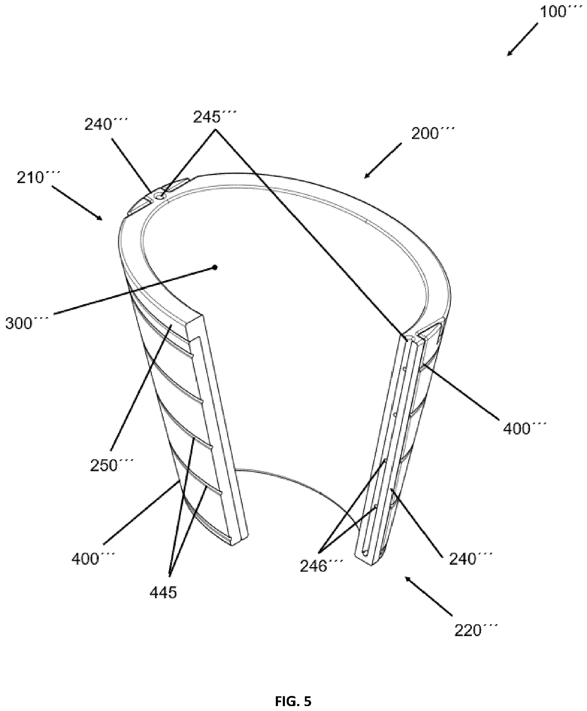

**Figur 5** zeigt eine Schnittdarstellung einer perspektivischen Seitenansicht einer weiteren Ausführungsform einer Augmentationsvorrichtung 100‴ in einem zusammengesetzten Zustand. Die Ausführungsform der Augmentationsvorrichtung 100‴ stimmt weitgehend mit der vorstehend beschriebenen und in den Figuren 1 bis 4 dargestellten Ausführungsformen überein, so dass zur Vermeidung von Wiederholungen auf die vorstehende Beschreibung verwiesen wird. Abwandlungen einer gegenüber den in den Figuren 1 bis 4 gezeigten Ausführungsform weisen dasselbe Bezugszeichen mit drei Apostrophen auf.

Die Ausführungsform der Augmentationsvorrichtung 100‴ unterscheidet sich von der Ausführungsform der Augmentationsvorrichtung 100" gemäß der Figur 4 darin, dass die Metallplatten 400‴ keine Sacklochbohrungen aufweisen, und dass die den Metallplatten 400‴zugewandten Stegleitungsmittelöffnungen 246‴ der Stegleitungsmittel 245‴ nicht fluidleitend mit an den Metallplatteninnenflächen verlaufenden inneren Nuten, sondern fluidleitend mit an der Metallplattenaußenfläche verlaufenden äußeren Nuten 445 verbunden ist. Die äußeren Nuten 445 ermöglichen eine möglichst weitgehende Verteilung eines in die Stegleitungsmittel 245‴ am proximalen Konusende 210‴ applizierten pharmazeutischen Fluids über den Umfang der Augmentationsvorrichtung 100‴.

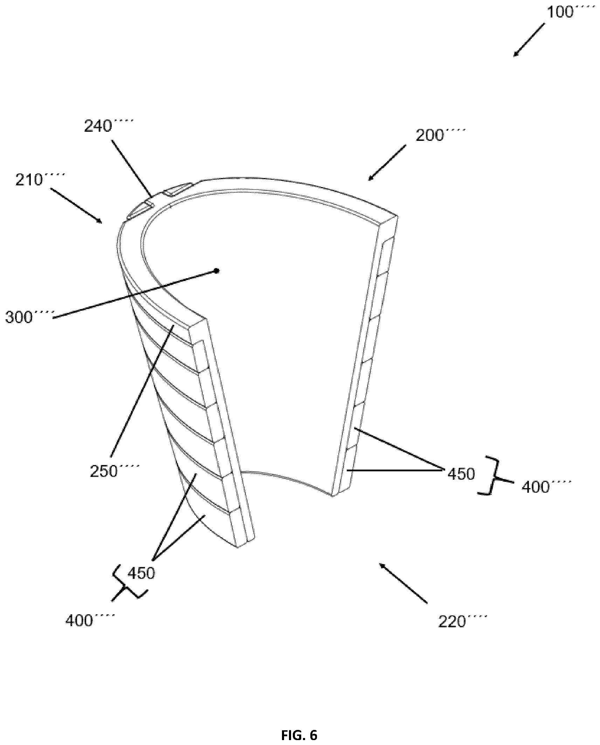

**Figur 6** zeigt eine Schnittdarstellung einer perspektivischen Seitenansicht einer weiteren Ausführungsform einer Augmentationsvorrichtung 100"" in einem zusammengesetzten Zustand. Die Ausführungsform der Augmentationsvorrichtung 100"" stimmt weitgehend mit der vorstehend beschriebenen und in den Figuren 1 bis 5 dargestellten Ausführungsformen überein, so dass zur Vermeidung von Wiederholungen auf die vorstehende Beschreibung verwiesen wird. Abwandlungen einer gegenüber den in den Figuren 1 bis 5 gezeigten Ausführungsform weisen dasselbe Bezugszeichen mit vier Apostrophen auf.

Die Ausführungsform der Augmentationsvorrichtung 100"" unterscheidet sich von den vorherigen Ausführungsformen dadurch, dass die Metallplatten 400"" auf beiden Seiten des Konus 200"" aus einer Vielzahl, insbesondere aus jeweils sechs, Metallplattensegmenten 450 (lediglich exemplarisch beziffert) gebildet sind. Die einzelnen Metallplattensegmente 450 sind reversibel trennbar voneinander, wodurch die Metallplatten 400"" schnell und einfach in ihrer axialen Erstreckung entlang der Längsachse der Augmentationsvorrichtung 100"" anpassbar sind. Beispielsweise können die beiden Metallplattensegmente 450 am distalen Konusende 220" entfernt werden, wodurch die Metallplatten 400"" um diese beiden Metallplattensegmente "gekürzt" werden. So kann schnell und einfach auf anatomische Begebenheiten eines Patienten im Zuge einer Operation eingegangen werden.

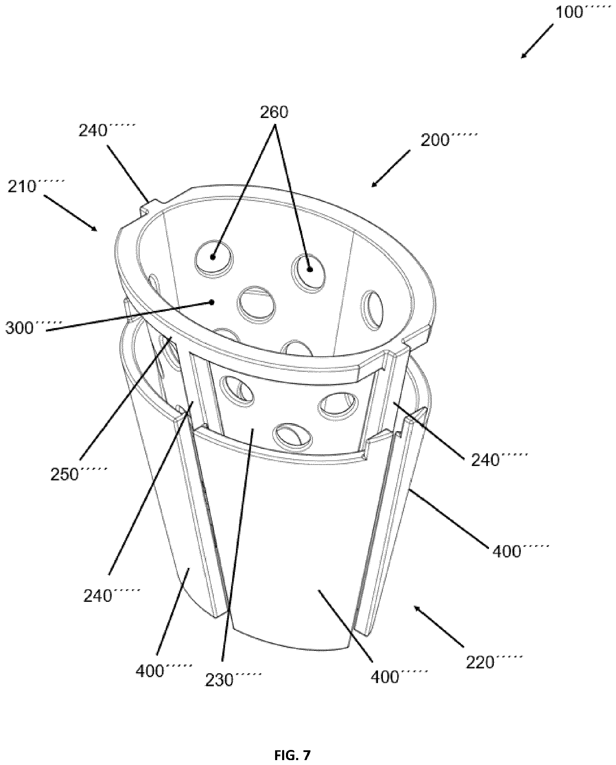

**Figur 7** zeigt eine perspektivische Seitenansicht einer weiteren Ausführungsform einer Augmentationsvorrichtung 100‴ʺ in einem getrennten Zustand. Die Ausführungsform der Augmentationsvorrichtung 100‴ʺ stimmt weitgehend mit der vorstehend beschriebenen und in den Figuren 1 bis 6 dargestellten Ausführungsformen überein, so dass zur Vermeidung von Wiederholungen auf die vorstehende Beschreibung verwiesen wird. Abwandlungen einer gegenüber den in den Figuren 1 bis 6 gezeigten Ausführungsform weisen dasselbe Bezugszeichen mit fünf Apostrophen auf.

Die Ausführungsform der Augmentationsvorrichtung 100"" unterscheidet sich von den vorherigen Ausführungsformen dadurch, dass der Konus 200‴ʺ vier im Wesentlichen gleichmäßig über die Mantelfläche 230‴ʺ verteilte axial verlaufende Stege 240‴ʺ und vier Metallplatten 400"" aufweist.

Der Konus 20‴ʺ weist Durchführungen 260 (lediglich exemplarisch beziffert) auf, welche im zusammengesetzten Zustand der Augmentationsvorrichtung 100‴ʺ den Kanal 300‴ʺ und die Metallplatten 400‴ʺ fluidleitend miteinander verbindet. Durch die Durchführungen 260 kann ein Knochenzementteig, welcher im implantierten Zustand der Augmentationsvorrichtung 100‴ʺ in den Kanal über das proximale Konusende 210‴ʺ appliziert wird, an die Metallplatten 400""' gelangen und etwaige Zwischenräume zwischen den Metallplatten 400‴ʺ und der Mantelfläche 230‴ʺ des Konus 200‴ʺ ausfüllen. Dies verringert Mikrobewegungen der Metallplatten 400‴ʺ und reduziert oder unterbindet einen Materialabrieb, insbesondere einen Metallabrieb.

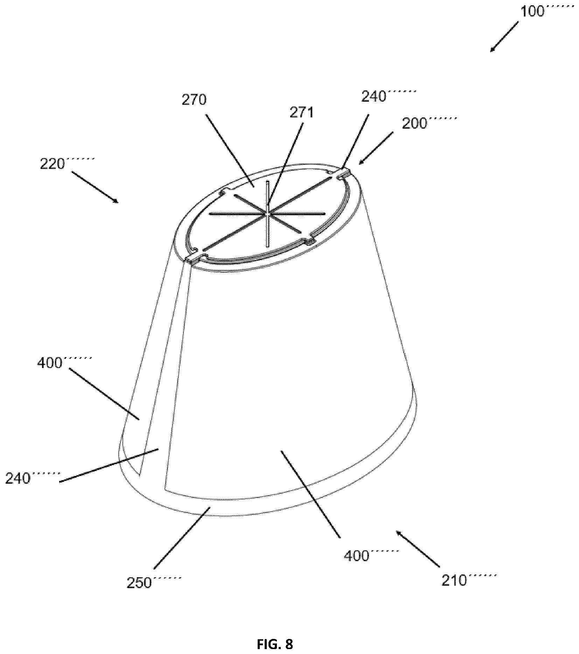

**Figur 8** zeigt eine perspektivische Seitenansicht einer weiteren Ausführungsform einer Augmentationsvorrichtung 100‴‴ in einem zusammengesetzten Zustand. Die Ausführungsform der Augmentationsvorrichtung 100""" stimmt weitgehend mit der vorstehend beschriebenen und in den Figuren 1 bis 7 dargestellten Ausführungsformen überein, so dass zur Vermeidung von Wiederholungen auf die vorstehende Beschreibung verwiesen wird.

Abwandlungen einer gegenüber den in den Figuren 1 bis 7 gezeigten Ausführungsform weisen dasselbe Bezugszeichen mit sechs Apostrophen auf.

Die Augmentationsvorrichtung 100""" weist am distalen Konusende 220‴‴ ein fest mit dem Konus 200‴‴ verbundenes Abstreifelement 270 auf, welches als elastisch verformbare Platte mit vier sich in einem Punkt schneidenden Einschnitten 271 (lediglich exemplarisch beziffert) ausgebildet ist. Die Einschnitte 271 bilden in der Platte des Abstreifelements 270 Plattenabschnitte aus. Das Abstreifelement 270 verschließt das distale Konusende 220‴‴ für einen durch das proximale Konusende 210‴‴ in den Kanal (nicht gezeigt in Figur 8; vgl. beispielsweise Figur 1) applizierten Knochenzementteig (nicht gezeigt) fluidleitend, so dass dieser nicht aus dem distalen Konusende 220‴‴ austritt und innerhalb des Kanals verbleibt. Das elastische Material des Abstreifelements 270 erlaubt im Zusammenspiel mit den Einschnitten 271, dass ein Schaft einer Prothese (nicht gezeigt) durch den Kanal aus Richtung des proximalen Konusende 210‴‴ durch den dort befindlichen Knochenzementteig und durch das Abstreifelement 270 aus dem distalen Konusende 220‴‴ herausgeschoben werden kann. Dabei werden die durch die Einschnitte 271 im Abstreifelement 270 ausgebildeten Plattenabschnitte durch den Schaft nach außen gebogen und streifen diesen beim Herausschieben aus dem distalen Konusende 220‴‴ von anhaftendem Knochenzementteig ab. Der Schaft ist somit beim Verlassen der Augmentationsvorrichtung 100""" am distalen Konusende 220‴‴ im Wesentlichen frei von Knochenzementteig.

### Bezugszeichen

- 100, 100', 100", 100‴,: Augmentationsvorrichtung
- 100"", 100‴ʺ, 100‴‴ 200, 200', 200", 200‴,: Konus
- 200"", 200‴ʺ, 200‴‴ 210, 210', 210", 210‴,: proximales Konusende
- 210ʺʺ, 210‴ʺ, 210‴‴ 220, 220', 220", 220‴,: distales Konusende
- 220"", 220‴ʺ, 220‴‴ 230, 230", 230‴ʺ: Mantelfläche
- 240, 240', 240", 240‴,: Steg
- 240"", 240‴ʺ, 240‴ʺ, 240a: Stegaußenfläche
- 245, 245', 245", 245‴: Stegleitungsmittel
- 246: Stegleitungsmittelöffnung
- 250, 250', 250", 250‴,: Schiebeelement
- 250"", 250‴ʺ, 250‴ʺʺ 260: Durchführung
- 270: Abstreifelement
- 271: Einschnitt
- 300, 300', 300", 300‴,: Kanal
- 300"", 300‴ʺ, 300‴‴ 400, 400', 400", 400‴,: Metallplatte
- 400"", 400‴ʺ, 400‴‴ 410, 410": Metallplatteninnenfläche
- 420, 420": Metallplattenaussparung
- 430, 430": Sacklochbohrung
- 440: innere Nut
- 445: äußere Nut
- 450: Metallplattensegment

## Patentansprüche

1. Augmentationsvorrichtung (100, 100', 100", 100‴, 100"", 100‴ʺ, 100‴‴) umfassend einen ringförmigen Konus (200, 200', 200", 200‴, 200"", 200‴ʺ, 200‴‴), der einen Kanal (300, 300', 300", 300‴, 300"", 300‴ʺ, 300‴‴) umgibt, welcher sich axial entlang einer Längsachse der Augmentationsvorrichtung (100, 100', 100", 100‴, 100ʺʺ, 100""', 100‴‴) von einem proximalen Konusende (210, 210', 210", 210‴, 210"", 210‴ʺ, 210‴‴) zu einem distalen Konusende (220, 220', 220", 220‴, 220"", 220‴ʺ, 220‴‴) erstreckt,
wobei ein Außendurchmesser des Konus (200, 200', 200", 200‴, 200"", 200‴ʺ, 200‴‴) vom proximalen Konusende (210, 210', 210", 210‴, 210"", 210""', 210‴‴) in Richtung des distalen Konusende (220, 220', 220", 220‴, 220ʺʺ, 220""', 220‴‴) abnimmt,
wobei auf einer Mantelfläche (230, 230", 230‴ʺ) des Konus (200, 200', 200", 200‴, 200"", 200‴ʺ, 200‴‴) mindestens zwei einer Außenkontur des Konus (200, 200', 200", 200‴, 200"", 200‴ʺ, 200‴‴) folgenden Metallplatten (400, 400', 400", 400‴, 400"", 400‴ʺ, 400‴‴) angeordnet sind,
**dadurch gekennzeichnet, dass**
der Konus (200, 200', 200", 200‴, 200"", 200‴ʺ, 200‴‴) von den Metallplatten (400, 400', 400", 400‴, 400"", 400‴ʺ, 400‴‴) durch Verschieben des Konus (200, 200', 200", 200‴, 200"", 200‴ʺ, 200‴‴) in Richtung des proximalen Konusende (210, 210', 210", 210‴, 210"", 210""', 210‴‴) reversibel lösbar ist.

2. Augmentationsvorrichtung (100, 100', 100", 100‴, 100"", 100""', 100‴‴) nach Anspruch 1, wobei die Metallplatten (400, 400', 400", 400‴, 400"", 400""', 400‴‴) durch axial verlaufende Spalte zwischen den Metallplatten (400, 400', 400", 400‴, 400"", 400‴ʺ, 400‴‴) voneinander beabstandet sind.

3. Augmentationsvorrichtung (100, 100', 100", 100‴, 100"", 100‴ʺ, 100‴‴) nach Anspruch 2, wobei an der Mantelfläche (230, 230", 230‴ʺ) des Konus (200, 200', 200", 200‴, 200"", 200‴ʺ, 200‴‴) axial verlaufende Stege (240, 240', 240", 240‴, 240"", 240‴ʺ, 240‴‴) ausgebildet sind, welche in die Spalte zwischen den Metallplatten (200, 200', 200", 200‴, 200"", 200""', 200‴‴) eingreifen.

4. Augmentationsvorrichtung (100, 100', 100", 100‴) nach Anspruch 3, wobei die Stege (240, 240', 240", 240‴) mindestens ein Stegleitungsmittel (245, 245', 245", 245‴) aufweisen, welche das proximale Konusende (210, 210', 210", 210‴, 210"", 210‴ʺ, 210‴‴) mit dem distalen Konusende (220, 220', 220", 220‴, 220"", 220""', 220‴‴) und/oder das proximale Konusende (210, 210', 210", 210‴, 210"", 210""', 210‴‴) mit einer Stegaußenfläche (240a) fluidleitend verbindet.

5. Augmentationsvorrichtung (100, 100', 100", 100‴, 100"", 100""', 100‴‴) nach einem der vorhergehenden Ansprüche, wobei der Konus (200, 200', 200", 200‴, 200"", 200‴ʺ, 200‴‴) mindestens ein Konusleitungsmittel aufweist, welche das proximale Konusende (210, 210', 210", 210‴, 210ʺʺ, 210""', 210‴‴) und das distale Konusende (220, 220', 220", 220‴, 220"", 220""', 220‴‴) fluidleitend verbindet.

6. Augmentationsvorrichtung (100, 100', 100", 100‴, 100"", 100""', 100‴‴) nach einem der vorhergehenden Ansprüche, wobei der Konus (200, 200', 200", 200‴, 200"", 200‴ʺ, 200‴‴) am proximalen Konusende (210, 210', 210", 210‴, 210"", 210‴ʺ, 210‴‴) ein Schiebeelement (250, 250', 250", 250‴, 250ʺʺ, 250‴ʺ, 250‴ʺʺ) aufweist, welches mit den Metallplatten (400, 400', 400", 400‴, 400"", 400‴ʺ, 400‴‴) an einem dem proximalen Konusende (210, 210', 210", 210‴, 210"", 210‴ʺ, 210‴‴) zugewandten proximalen Metallplattenende derart zusammenwirkt, dass eine Krafteinwirkung auf den Konus (200, 200', 200", 200‴, 200"", 200""', 200‴‴) aus Richtung des proximalen Konusende (210, 210', 210", 210‴, 210"", 210""', 210‴‴) über das Schiebeelement (250, 250', 250", 250‴, 250ʺʺ, 250‴ʺ, 250‴ʺʺ) auf die Metallplatten (400, 400', 400", 400‴, 400"", 400""', 400‴‴) übertragbar ist.

7. Augmentationsvorrichtung (100, 100', 100", 100‴, 100"", 100""', 100‴‴) nach einem der vorhergehenden Ansprüche, wobei die Metallplatten (400, 400', 400", 400‴, 400"", 400‴ʺ, 400‴‴) derart an der Mantelfläche (230, 230", 230""') des Konus (200, 200', 200", 200‴, 200"", 200‴ʺ, 200‴‴) angeordnet sind, dass zwischen den Metallplatten (400, 400', 400", 400‴, 400"", 400""', 400‴‴) und der Mantelfläche (230, 230", 230‴ʺ) eine reversibel formschlüssige Verbindung ausgebildet ist.

8. Augmentationsvorrichtung (100, 100', 100", 100‴, 100"", 100""', 100‴‴) nach einem der vorhergehenden Ansprüche, wobei eine Konuswanddicke des Konus (200, 200', 200", 200‴, 200"", 200‴ʺ, 200‴‴) größer oder gleich einer Metallplattenwanddicke der Metallplatten (400, 400', 400", 400‴, 400"", 400‴ʺ, 400‴‴) ist.

9. Augmentationsvorrichtung (100, 100', 100", 100‴, 100ʺʺ, 100""', 100‴‴) nach einem der vorhergehenden Ansprüche, wobei die Metallplatten (400, 400', 400", 400‴, 400"", 400‴ʺ, 400‴‴) mindestens 80 Flächen-% der Mantelfläche (230, 230", 230""') des Konus (200, 200', 200", 200‴, 200"", 200‴ʺ, 200‴‴) bedecken.

10. Augmentationsvorrichtung (100', 100") nach einem der vorhergehenden Ansprüche, wobei eine der Mantelfläche (230") des Konus (200', 200") abgewandte Metallplattenaußenfläche aufgeraut ist, porös ist und/oder Sacklochbohrungen (430, 430") aufweist.

11. Augmentationsvorrichtung (100', 100") nach Anspruch 10, wobei die Sacklochbohrungen (430, 430") einen Durchmesser von kleiner 3 mm aufweisen.

12. Augmentationsvorrichtung (100‴ʺ) nach einem der vorhergehenden Ansprüche, wobei der Konus (200‴ʺ) Durchführungen (260) aufweist, welche den Kanal (300‴ʺ) mit einem Zwischenraum zwischen dem Konus (200""') und den Metallplatten (400‴ʺ) fluidleitend verbinden.

13. Augmentationsvorrichtung (100‴‴) nach einem der vorhergehenden Ansprüche, wobei an einem dem distalen Konusende (220‴‴) zugewandten distalen Kanalende ein Abstreifelement (270) angeordnet ist.

14. Augmentationsvorrichtung (100, 100', 100", 100‴, 100"", 100""', 100‴‴) nach einem der vorhergehenden Ansprüche, wobei die Metallplatten (400, 400', 400", 400‴, 400"", 400‴ʺ, 400‴‴) ein biokompatibles Metall umfassen.

15. Augmentationsvorrichtung (100, 100', 100", 100‴, 100"", 100""', 100‴‴) nach einem der vorhergehenden Ansprüche, wobei der Konus (200, 200', 200", 200‴, 200"", 200‴ʺ, 200‴‴) ein biokompatibles Polymer umfassen.

## Claims

1. An augmentation device (100, 100', 100", 100‴, 100"", 100 , 100""") comprising an annular cone (200, 200', 200", 200‴, 200"", 200‴ʺ, 200‴‴) surrounding a channel (300, 300', 300", 300‴, 300"", 300‴ʺ, 300"""), which extends axially along a longitudinal axis of the augmentation device (100, 100', 100", 100‴, 100"", 100‴ʺ, 100‴‴) from a proximal cone end (210, 210', 210", 210‴, 210"", 210‴ʺ, 210‴‴) to a distal cone end (220, 220', 220", 220‴, 220"", 220‴ʺ, 220‴‴),
an outer diameter of the cone (200, 200', 200", 200‴, 200"", 200""', 200‴‴) decreasing from the proximal cone end (210, 210', 210", 210‴, 210"", 210‴ʺ, 210‴‴) in the direction of the distal cone end (220, 220', 220", 220‴, 220"", 220""', 220"""),
at least two metal plates (400, 400', 400", 400‴, 400"", 400""', 400‴‴) following an outer contour of the cone (200, 200', 200", 200‴, 200"", 200""', 200‴‴) being arranged on a lateral surface (230, 230", 230""') of the cone (200, 200', 200", 200‴, 200"", 200""', 200‴‴),
**characterized in that** the cone (200, 200', 200", 200‴, 200"", 200‴ʺ, 200‴‴) is reversibly detachable from the metal plates (400, 400', 400", 400‴, 400"", 400‴ʺ, 400‴‴) by displacing the cone (200, 200', 200", 200‴, 200"", 200‴ʺ, 200‴‴) in the direction of the proximal cone end (210, 210', 210", 210‴, 210"", 210""', 210""").

2. The augmentation device (100, 100', 100", 100‴, 100"", 100""', 100‴‴) according to claim 1, wherein the metal plates (400, 400', 400", 400‴, 400ʺʺ, 400‴ʺ, 400‴‴) are spaced apart from one another by axially extending gaps between the metal plates (400, 400', 400", 400‴, 400"", 400‴ʺ, 400‴‴).

3. The augmentation device (100, 100', 100", 100‴, 100"", 100""', 100‴‴) according to claim 2, wherein axially extending projections (240, 240', 240", 240‴, 240"", 240‴ʺ, 240‴‴) are formed on the lateral surface (230, 230", 230""') of the cone (200, 200', 200", 200‴, 200"", 200""', 200‴‴), which engage in the gap between the metal plates (200, 200', 200", 200‴, 200"", 200‴ʺ, 200‴‴).

4. The augmentation device (100, 100', 100", 100‴) according to claim 3, wherein the projections (240, 240', 240", 240‴) comprise at least one projection guiding means (245, 245', 245", 245‴) which connects the proximal cone end (210, 210', 210", 210‴, 210"", 210‴ʺ, 210‴‴) to the distal cone end (220, 220', 220", 220‴, 220"", 220‴ʺ, 220‴‴) and/or the proximal cone end (210, 210', 210", 210‴, 210"", 210""', 210""") to a projection outer surface (240a) in a fluid-conducting manner.

5. The augmentation device (100, 100', 100", 100‴, 100"", 100‴ʺ, 100""") according to any of the preceding claims, wherein the cone (200, 200', 200", 200‴, 200"", 200 , 200‴‴) comprises at least one cone guiding means which connects the proximal cone end (210, 210', 210", 210‴, 210"", 210""', 210‴‴) and the distal cone end (220, 220', 220", 220‴, 220"", 220‴ʺ, 220‴‴) in a fluid-conducting manner.

6. The augmentation device (100, 100', 100", 100‴, 100"", 100""', 100‴‴) according to any of the preceding claims, wherein the cone (200, 200', 200", 200‴, 200"", 200 , 200‴‴) comprises, at the proximal cone end (210, 210', 210", 210‴, 210ʺʺ, 210""', 210‴‴), a sliding element (250, 250', 250", 250‴, 250"", 250""', 250‴‴) which interacts with the metal plates (400, 400', 400", 400‴, 400"", 400‴ʺ, 400‴‴) at one proximal metal plate end facing the proximal cone end (210, 210', 210", 210‴, 210"", 210""', 210‴‴) such that a force acting on the cone (200, 200', 200", 200‴, 200"", 200 , 200""") from the direction of the proximal cone end (210, 210', 210", 210‴, 210"", 210""', 210""") can be transferred to the metal plates (400, 400', 400", 400‴, 400"", 400""', 400""") via the sliding element (250, 250', 250", 250‴, 250"", 250""', 250‴‴).

7. The augmentation device (100, 100', 100", 100‴, 100"", 100""', 100""") according to any of the preceding claims, wherein the metal plates (400, 400', 400", 400‴, 400"", 400‴ʺ, 400""") are arranged on the lateral surface (230, 230", 230""') of the cone (200, 200', 200", 200‴, 200"", 200""', 200‴‴) such that a reversible form-fitting connection is formed between the metal plates (400, 400', 400", 400‴, 400"", 400‴ʺ, 400‴‴) and the lateral surface (230, 230", 230‴ʺ).

8. The augmentation device (100, 100', 100", 100‴, 100"", 100""', 100""") according to any of the preceding claims, wherein a cone wall thickness of the cone (200, 200', 200", 200‴, 200"", 200‴ʺ, 200‴‴) is greater than or equal to a metal plate wall thickness of the metal plates (400, 400', 400", 400‴, 400"", 400‴ʺ, 400‴‴).

9. The augmentation device (100, 100', 100", 100‴, 100"", 100""', 100‴‴) according to any of the preceding claims, wherein the metal plates (400, 400', 400", 400‴, 400"", 400‴ʺ, 400""") cover at least 80% by area of the lateral surface (230, 230", 230""') of the cone (200, 200', 200", 200‴, 200"", 200""', 200‴‴).

10. The augmentation device (100', 100") according to any of the preceding claims, wherein a metal plate outer surface facing away from the lateral surface (230") of the cone (200', 200") is roughened, is porous and/or comprises blind holes (430, 430").

11. The augmentation device (100', 100") according to claim 10, wherein the blind holes (430, 430") have a diameter of less than 3 mm.

12. The augmentation device (100‴ʺ) according to any of the preceding claims, wherein the cone (200""') has feedthroughs (260) which connect the channel (300‴ʺ) to an intermediate space between the cone (200‴ʺ) and the metal plates (400‴ʺ) in a fluid-conducting manner.

13. The augmentation device (100‴‴) according to any of the preceding claims, wherein a wiper element (270) is arranged at a distal channel end facing the distal cone end (220‴‴).

14. The augmentation device (100, 100', 100", 100‴, 100"", 100‴ʺ, 100‴‴) according to any of the preceding claims, wherein the metal plates (400, 400', 400", 400‴, 400"", 400‴ʺ, 400‴‴) comprise a biocompatible metal.

15. The augmentation device (100, 100', 100", 100‴, 100"", 100‴ʺ, 100‴‴) according to any of the preceding claims, wherein the cone (200, 200', 200", 200‴, 200"", 200‴ʺ, 200‴‴) comprises a biocompatible polymer.

## Revendications

1. Dispositif d'augmentation (100, 100', 100", 100‴, 100"", 100‴ʺ, 100""") comprenant un cône (200, 200', 200", 200‴, 200"", 200‴ʺ, 200""") annulaire qui entoure un canal (300, 300', 300", 300‴, 300"", 300‴ʺ, 300"""), lequel s'étend axialement le long d'un axe longitudinal du dispositif d'augmentation (100, 100', 100", 100‴, 100"", 100‴ʺ, 100"""), d'une extrémité de cône proximale (210, 210', 210", 210‴, 210"", 210‴ʺ, 210""") à une extrémité de cône distale (220, 220', 220", 220‴, 220"", 220‴ʺ, 220"""),
dans lequel un diamètre extérieur du cône (200, 200', 200", 200‴, 200"", 200‴ʺ, 200""") diminue depuis l'extrémité de cône proximale (210, 210', 210", 210‴, 210"", 210‴ʺ, 210""") en direction de l'extrémité de cône distale (220, 220', 220", 220‴, 220"", 220‴ʺ, 220"""), dans lequel au moins deux plaques métalliques (400, 400', 400", 400‴, 400"", 400‴ʺ, 400""") suivant un contour extérieur du cône (200, 200', 200", 200‴, 200"", 200‴ʺ, 200""") sont agencées sur une surface périphérique (230, 230", 230‴ʺ) du cône (200, 200', 200", 200‴, 200"", 200‴ʺ, 200"""),
**caractérisé en ce que** le cône (200, 200', 200", 200‴, 200"", 200‴ʺ, 200""") peut être détaché de manière réversible des plaques métalliques (400, 400', 400", 400‴, 400"", 400‴ʺ, 400""") par un déplacement du cône (200, 200', 200", 200‴, 200"", 200‴ʺ, 200""") en direction de l'extrémité de cône proximale (210, 210', 210", 210‴, 210"", 210‴ʺ, 210""").

2. Dispositif d'augmentation (100, 100', 100", 100‴, 100"", 100‴ʺ, 100""") selon la revendication 1, dans lequel les plaques métalliques (400, 400', 400", 400‴, 400"", 400‴ʺ, 400""") sont espacées les unes des autres par des fentes évoluant axialement entre les plaques métalliques (400, 400', 400", 400‴, 400"", 400‴ʺ, 400""").

3. Dispositif d'augmentation (100, 100', 100", 100‴, 100"", 100‴ʺ, 100""") selon la revendication 2, dans lequel des entretoises (240, 240', 240", 240‴, 240"", 240‴ʺ, 240""") évoluant axialement sont formées sur la surface périphérique (230, 230", 230‴") du cône (200, 200', 200", 200‴, 200"", 200‴ʺ, 200"""), lesquelles entretoises viennent en prise dans les fentes entre les plaques métalliques (200, 200', 200", 200‴, 200"", 200‴ʺ, 200""").

4. Dispositif d'augmentation (100, 100', 100", 100‴) selon la revendication 3, dans lequel les entretoises (240, 240', 240", 240‴) présentent au moins un moyen de guidage d'entretoise (245, 245', 245", 245‴), lequel relie de manière conductrice de fluide l'extrémité de cône proximale (210, 210', 210", 210"', 210"", 210‴ʺ, 210""") à l'extrémité de cône distale (220, 220', 220", 220‴, 220"", 220‴ʺ, 220""") et/ou l'extrémité de cône proximale (210, 210', 210", 210‴, 210"", 210‴ʺ, 210""") à une surface extérieure d'entretoise (240a).

5. Dispositif d'augmentation (100, 100', 100", 100‴, 100"", 100‴ʺ, 100""") selon l'une des revendications précédentes, dans lequel le cône (200, 200', 200", 200‴, 200"", 200‴ʺ, 200""") présente au moins un moyen de guidage de cône, lequel relie de manière conductrice de fluide l'extrémité de cône proximale (210, 210', 210", 210‴, 210"", 210‴ʺ, 210""") et l'extrémité de cône distale (220, 220', 220", 220‴, 220"", 220‴ʺ, 220""").

6. Dispositif d'augmentation (100, 100', 100", 100‴, 100"", 100‴ʺ, 100""") selon l'une des revendications précédentes, dans lequel le cône (200, 200', 200", 200‴, 200"", 200‴ʺ, 200""") présente, au niveau de l'extrémité de cône proximale (210, 210', 210", 210‴, 210"", 210‴ʺ, 210"""), un élément coulissant (250, 250', 250", 250‴, 250"", 250‴ʺ, 250"""), lequel coopère avec les plaques métalliques (400, 400', 400", 400‴, 400"", 400‴ʺ, 400""") au niveau d'une extrémité de plaque métallique proximale faisant face à l'extrémité de cône proximale (210, 210', 210", 210‴, 210"", 210‴ʺ, 210"""), de telle sorte qu'une force exercée sur le cône (200, 200', 200", 200‴, 200"", 200‴ʺ, 200""") provenant de la direction de l'extrémité de cône proximale (210, 210', 210", 210‴, 210"", 210‴ʺ, 210""") peut être transférée aux plaques métalliques (400, 400', 400", 400‴, 400"", 400‴ʺ, 400""") par l'intermédiaire de l'élément coulissant (250, 250', 250", 250‴, 250"", 250‴ʺ, 250""").

7. Dispositif d'augmentation (100, 100', 100", 100‴, 100"", 100‴ʺ, 100""") selon l'une des revendications précédentes, dans lequel les plaques métalliques (400, 400', 400", 400‴, 400"", 400‴ʺ, 400""") sont agencées sur la surface périphérique (230, 230", 230‴ʺ) du cône (200, 200', 200", 200‴, 200"", 200‴ʺ, 200""") de telle sorte qu'une liaison par complémentarité de forme réversible est formée entre les plaques métalliques (400, 400', 400", 400‴, 400"", 400‴ʺ, 400""") et la surface périphérique (230, 230", 230‴ʺ).

8. Dispositif d'augmentation (100, 100', 100", 100‴, 100"", 100‴ʺ, 100""") selon l'une des revendications précédentes, dans lequel une épaisseur de paroi de cône du cône (200, 200', 200", 200‴, 200"", 200‴ʺ, 200""") est supérieure ou égale à une épaisseur de paroi de plaque métallique des plaques métalliques (400, 400', 400", 400‴, 400"", 400‴ʺ, 400""").

9. Dispositif d'augmentation (100, 100', 100", 100‴, 100"", 100‴ʺ, 100""") selon l'une des revendications précédentes, dans lequel les plaques métalliques (400, 400', 400", 400‴, 400"", 400‴ʺ, 400""") recouvrent au moins 80 % de surface de la surface périphérique (230, 230", 230‴ʺ) du cône (200, 200', 200", 200‴, 200"", 200‴ʺ, 200""").

10. Dispositif d'augmentation (100', 100") selon l'une des revendications précédentes, dans lequel une surface extérieure de plaque métallique opposée à la surface périphérique (230") du cône (200', 200") est rendue rugueuse, est poreuse et/ou présente des trous borgnes (430, 430").

11. Dispositif d'augmentation (100', 100") selon la revendication 10, dans lequel les trous borgnes (430, 430") présentent un diamètre inférieur à 3 mm.

12. Dispositif d'augmentation (100‴ʺ) selon l'une des revendications précédentes, dans lequel le cône (200‴ʺ) présente des traversées (260), lesquelles relient de manière conductrice de fluide le canal (300‴ʺ) à un espace intermédiaire entre le cône (200‴ʺ) et les plaques métalliques (400‴ʺ).

13. Dispositif d'augmentation (100""") selon l'une des revendications précédentes, dans lequel un élément de raclage (270) est agencé au niveau d'une extrémité de canal distale faisant face à l'extrémité de cône distale (220""").

14. Dispositif d'augmentation (100, 100', 100", 100‴, 100"", 100‴ʺ, 100""") selon l'une des revendications précédentes, dans lequel les plaques métalliques (400, 400', 400", 400‴, 400"", 400‴ʺ, 400""") comprennent un métal biocompatible.

15. Dispositif d'augmentation (100, 100', 100", 100‴, 100"", 100‴ʺ, 100""") selon l'une des revendications précédentes, dans lequel le cône (200, 200', 200", 200‴, 200"", 200‴ʺ, 200""") comprend un polymère biocompatible.
